# EUROPÄISCHE PATENTSCHRIFT

(11) **EP 1 857 252 B1**
(45) Veröffentlichungstag und Bekanntmachung des Hinweises auf die Patenterteilung: **21.08.2019**
(21) Anmeldenummer: 07009566.6
(22) Anmeldetag: 12.05.2007
(51) Int. Cl.: B29C 45/16, A61M 39/04, A61M 39/12, C08L 23/08, C08L 23/10, B29L 31/00, A61M 39/10

(54) **Medizinische Baugruppe**
Medical assembly
Ensemble médical

(30) Priorität: 18.05.2006 DE 102006023301
(43) Veröffentlichungstag der Anmeldung: 21.11.2007
(73) Patentinhaber: RAUMEDIC AG, 95213 Münchberg (DE)
(72) Erfinder: Jakob, Thomas, 95100 Selb (DE); Täubert, Markus, 95111 Rehau (DE); Bucher, Jochen, 95111 Rehau (DE)
(74) Vertreter: Rau, Schneck & Hübner Patentanwälte Rechtsanwälte PartGmbB

(56) Entgegenhaltungen:
- EP-A- 0 999 146
- US-A- 4 664 659
- US-A- 4 665 959
- US-A- 5 641 184
- US-A1- 2005 238 830

## Beschreibung

Die Erfindung betrifft eine medizinische Baugruppe nach dem Oberbegriff des Anspruchs 1.

Die US 5,641,184 offenbart ein Schlauchstück für medizinische Zwecke mit einem elastischen Einsatz, der Durchstecheigenschaften aufweist.

Die US 4,665,959 offenbart eine Steckanordnung, bei der ein flexibles Material mit Durchstecheigenschaften in einem Kunststoffkörper eingebettet ist.

Die EP 0 999 146 A2 offenbart einen durchstechbaren Verschluss für einen Behälter.

Die US 4,664,659 offenbart einen Beutel mit einem daran angeformten Schlauch und Nadel.

Die US 2005/0238830 A1 offenbart Polymerverbindungen, die Cycloolefin-Copolymere aufweisen.

Die US 2004/0133169 A1 offenbart einen Spritzenzylinder, auf den ein Verbindungsabschnitt aufgeschweißt ist.

Aus der US 2005/02 34 407 A1 ist eine Komponente einer medizinischen Baugruppe zur Fluidförderung beschrieben, bei der ein Ventilkörper und ein Grundabschnitt aus Kunststoffmaterial ausgestaltet und zusammengegossen sind. Die EP 0 556 034 A1 beschreibt verschiedene Komponenten eines Infusionssystems, bei dessen Herstellung Kunststoff-Zusammensetzungen miteinander laminiert werden.

Die WO 02/053 360 A2 beschreibt einen Injektionsport, bei dem eine Haftverbindung zwischen einem Grundkörper und einem Verschlusskörper einerseits und zwischen dem Grundkörper und einem diesen umgebenden Schlauchabschnitt andererseits durch die Wahl einander jeweils zugewandter Lagen des mehrschichtigen Grundkörpers, des Schlauchabschnitts sowie des Verschlusskörpers erreicht werden. Die WO 02/066 595 A1 beschreibt einen Injektionsport mit einem Grundkörper und einem Verschlusskörper. Der Grundkörper kann insbesondere aus Polyethylen sein. Ein weiteres Infusionsabgabeset ist bekannt aus der DE 600 18 771 T2.

Ein medizinisches Arbeitsmittel sowie eine medizinische Baugruppe, die ein derartiges Arbeitsmittel verwendet, sind ferner durch offenkundige Vorbenutzung bekannt. Derartige Arbeitsmittel müssen folgende Anforderungen erfüllen: Der Verschlusskörper muss sicher mit dem Grundkörper verbunden sein, so dass ein dauerhaft dichtender Verschluss der Zugangsöffnung sichergestellt ist. Der Verschlusskörper muss zudem Durchstecheigenschaften aufweisen, die zum Beispiel entsprechend der DIN-Norm 58362 und 58363-15 geprüft werden können. Es muss also sichergestellt sein, dass nach dem Durchstechen des Verschlusskörpers zum Beispiel mit einer Injektionsnadel und dem Herausziehen der Nadel aus dem Verschlusskörper letzterer die Zugangsöffnung im Grundkörper wieder vollkommen abdichtet. Bei den aus dem Stand der Technik bekannten Arbeitsmitteln konnten diese Anforderungen nur mit hohem Herstellungsaufwand bzw. durch Einsatz von biologisch bedenklichen Materialien, wie z. B. Latex, für den Verschlusskörper erfüllt werden. Soweit die vorbekannten Arbeitsmittel eine Montage des Verschlusskörpers im Grundkörper erforderten, war dieser Montageschritt eine zusätzliche Fehlerquelle.

Es ist daher eine Aufgabe der vorliegenden Erfindung, eine medizinische Baugruppe der eingangs genannten Art derart weiterzubilden, dass dessen Herstellungsaufwand reduziert wird.

Diese Aufgabe ist erfindungsgemäß gelöst durch eine medizinische Baugruppe mit den im Kennzeichnungsteil des Anspruches 1 angegebenen Merkmalen.

Durch die Herstellung des Grundkörpers mit dem Verschlusskörper in Mehrkomponenten-Spritzgußtechnik resultiert ein kostengünstiges medizinisches Arbeitsmittel. Die beiden Kunststoffmaterialien des Grundkörpers einerseits und des Verschlusskörpers andererseits können dabei ohne weiteres so aufeinander abgestimmt werden, dass der Verschlusskörper in der Zugangsöffnung des Grundkörpers haftet. Der Begriff "einheitliches Kunststoffmaterial" bedeutet im Sinne dieser Anmeldung, dass der Grundkörper einerseits und der Verschlusskörper andererseits im Verbindungsbereich keine Materialbeschichtungen aufweisen, sondern dort aus einheitlichen, homogenen Materialien gefertigt sind. Eine Beschichtung des Grundkörpers beabstandet zum Anbringungsort des Verschlusskörpers hat hingegen auf die Einheitlichkeit des Kunststoffmaterials im Sinne der Anmeldung keinen Einfluss. Auch ein derart beschichteter Grundkörper ist ein Grundkörper aus einem einheitlichen Kunststoffmaterial.

Bevorzugte Verwendungen des medizinischen Arbeitsmittels sind die Verwendung als Injektionsport oder die Verwendung als Stufenkonnektor. Bei diesen Verwendungen kommen die Vorteile, die im Zusammenhang mit den Ansprüchen erläutert werden, besonders gut zum Tragen.

Die Ausgestaltung des medizinischen Arbeitsmittels derart, dass das erste Kunststoffmaterial so auf das dritte Kunststoffmaterial abstimmbar ist, dass die Haftverbindung zwischen dem Grundkörper und dem Schlauchabschnitt durch Erwärmung von diesen auf eine Temperatur unter 160°C entsteht, sorgt für eine haftende und dichte Verbindung des Grundkörpers mit einem auf den Grundkörper aufgeschobenen Verbindungsabschnitt, z.B. mit einem Schlauchabschnitt. Dabei kann auf eine aufwändige Kunststoff-Schweißverbindung verzichtet werden.

Es wurde gefunden, dass sich durchaus Kunststoffmaterialien für den Grundkörper, den Verschlusskörper und den Verbindungsabschnitt angeben lassen, die die eingangs erwähnten Anforderungen erfüllen, ohne dass es erforderlich ist, den Grundkörper mit dem Verbindungsabschnitt über ein aufwändiges Kunststoff-Schweißverfahren zu verbinden. Erfindungsgemäß kann der Schlauchabschnitt aus dem dritten Kunststoffmaterial mit dem Grundkörper aus dem ersten Kunststoffmaterial im Rahmen einer bei dem medizinischen Arbeitsmittel in der Regel sowieso erforderlichen Dampfsterilisation verbunden werden. Bei der Dampfsterilisation werden Temperaturen zum Beispiel von 134°C oder von 121°C erreicht, wobei das erste und das dritte Kunststoffmaterial so ausgelegt sein können, dass bei diesen Temperaturen und den üblichen Sterilisationszeiten eine haftende und dichte Verbindung des Schlauchabschnitts am Hülsenabschnitt des Grundkörpers resultiert. Aufgrund der Haftverbindung des Grundkörpers am Verschlusskörper entfällt die Notwendigkeit, den Verschlusskörper aufwändig am Grundkörper zu montieren. Insgesamt resultiert ein mit geringem Aufwand herstellbares Arbeitsmittel.

Eine Ausgestaltung des Arbeitsmittels als Mehrkomponenten-Spritzgussteil führt zu einer unaufwändigen Herstellung, insbesondere in einem Massenproduktionsverfahren.

Eine Mischung aus Polypropylen (PP) und einem Cycloolefin-Polymer (COP) als erstes Kunststoffmaterial mit einem Gewichtsprozentverhältnis PP/COP zwischen 90:10 und 10:90 hat sich für eine Abstimmung des ersten Kunststoffmaterials auf das zweite Kunststoffmaterial zur Verbindung mit dem Verschlusskörper einerseits und auf das dritte Kunststoffmaterial zur Verbindung mit dem Schlauchabschnitt andererseits als besonders vorteilhaft herausgestellt. Untersuchungen der Anmelderin haben ergeben, dass mit steigendem PP-Anteil die Haftfestigkeit des Grundkörpers am Verschlusskörper steigt. Mit steigendem COP-Anteil steigt wiederum die Haftfestigkeit des Grundkörpers am Schlauchabschnitt.

Ein Schlauchabschnitt umfassend ein Acetat-oder Acrylat-Polymer hat ein hinsichtlich seiner Materialeigenschaften bevorzugt eingesetztes Schlauchmaterial im medizinischen Bereich. Ein derartiger Schlauchabschnitt lässt sich mit dem Grundkörper durch Dampfsterilisation verbinden.

Gewichtsprozentverhältnisse nach Anspruch 2 führen je nach der spezifischen Geometrie des Arbeitsmittels und den Anforderungen an die Verbindungsfestigkeit des Verschlusskörpers am Grundkörper einerseits und des Schlauchabschnitts am Grundkörper andererseits zu einer optimalen Abstimmung der erforderlichen Materialparameter.

Eine Innenwandgestaltung nach Anspruch 3 führt dazu, dass keine unerwünschte Ausrichtung von Molekülketten des zweiten Kunststoffmaterials längs des Verlaufs der Zugangsöffnung erfolgt. Wie Untersuchungen der Anmelderin ergeben haben, führt eine derartige unerwünschte Ausrichtung zu einer Herabsetzung der Durchstecheigenschaften des Verschlusskörpers. Dadurch, dass der Querschnitt der Innenwand dort, wo der Verschlusskörper an dieser anliegt, im Wesentlichen konstant bleibt, wird eine Ausrichtung von Molekülketten, die sich in Querschnittsverengungen bevorzugt einstellt, vermieden. Eine geringfügige Querschnittsverengung, zum Beispiel durch Einsatz einzelner Formschluss-Sicherungskörper, beeinträchtigt die Durchstecheigenschaften kaum, so dass derartige Formschlusskörper im Einklang mit der Forderung nach einem im Wesentlichen konstanten Querschnitt der Innenwand stehen.

Eine Innenwandgestaltung nach Anspruch 4 vermeidet eine unerwünschte Ausrichtung von Molekülketten des zweiten Kunststoffmaterials praktisch vollständig.

Eine Ausgestaltung des Arbeitsmittels als Mehrkomponenten-Spritzgussteil führt zu einer unaufwändigen Herstellung, insbesondere in einem Massenproduktionsverfahren.

Eine Anordnung des Verschlusskörpers nach Anspruch 5 erleichtert das Aufsetzen einer Injektionsnadel auf den Verschlusskörper, da diese praktisch nicht vom Arbeitsmittel abrutschen kann.

Ausführungsbeispiele der Erfindung werden nachfolgend anhand der Zeichnung näher erläutert. In dieser zeigen:
- Fig. 1: Eine Seitenansicht eines medizinischen Arbeitsmittels in Form eines Injektionsports;
- Fig. 2: einen axialen Längsschnitt durch einen Grundkörper des medizinischen Arbeitsmittels nach Fig. 1;
- Fig. 3: eine zu Fig. 2 ähnliche Darstellung einer medizinischen Baugruppe mit einem medizinischen Arbeitsmittel nach Fig. 1, welches neben dem Grundkörper noch einen Verschlusskörper aufweist, und einem Schlauchabschnitt; und
- Fig. 4 bis 6: schematisch Ausschnitte weiterer Ausführungsformen medizinischer Arbeitsmittel jeweils in der Umgebung des Verschlusskörpers;
- Fig. 7: eine perspektivische Ansicht einer weiteren Ausführungsform eines medizinischen Arbeitsmittels in Form eines Stufenkonnektors.

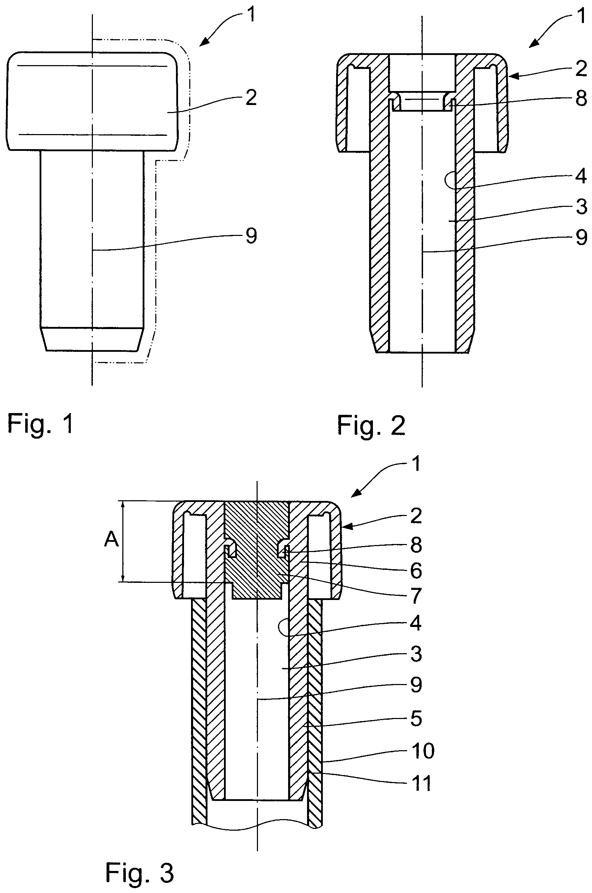

Die Fig. 1 bis 3 zeigen eine erste Ausführungsform eines medizinischen Arbeitsmittels in Form eines Injektionsports 1. Der Injektionsport 1 ermöglicht in montiertem Zustand die Zugabe beispielsweise eines Medikaments mit Hilfe einer Injektionsnadel, die den Injektionsport 1 definiert durchsticht.

Der Injektionsport 1 hat einen in der Fig. 2 dargestellten Grundkörper 2 aus einem ersten Kunststoffmaterial. Hierbei handelt es sich um eine Mischung bzw. einen Blend aus Polypropylen (PP) mit einem Cycloolefin-Polymer (COP). Es kann auch ein Cycloolefin-Copolymer (COC) als Cycloolefin-Polymer eingesetzt werden. Das Gewichtsprozentverhältnis PP/COP beträgt beim Grundkörper 2 etwa 50:50. Auch andere GewichtsprozentVerhältnisse PP/COP zwischen 90:10 und 10:90 sind möglich, vorzugsweise zwischen 80:20 und 20:80, mehr bevorzugt zwischen 70:30 und 30:70, noch mehr bevorzugt zwischen 60:40 und 40:60.

Der Grundkörper 2 hat eine Zugangsöffnung 3 mit einer Innenwand 4. Ferner hat der Grundkörper 2 einen Hülsenabschnitt 5. Der Grundkörper 2 ist um die Längsachse 9 rotationssymmetrisch.

Im Bereich eines Kopfabschnitts 6, der einstückig in den Hülsenabschnitt 5 übergeht, ist die Zugangsöffnung 3 von einem Verschlusskörper 7 in Form eines Stopfens bzw. Septums verschlossen. Der Verschlusskörper 7 ist aus einem zweiten Kunststoffmaterial, nämlich aus TPE (thermoplastisches Elastomer). Das zweite Kunststoffmaterial hat im Vergleich zum ersten Kunststoffmaterial des Grundkörpers 2 eine geringere Shorehärte. Der Verschlusskörper 7 haftet stoffschlüssig an der Innenwand 4 der Zugangsöffnung 3 des Grundkörpers 2 an. Bei der Ausführungsform der Fig. 1 bis 3 ist zur Sicherung der Haftung des Verschlusskörpers 7 am Grundkörper 2 einstückig innen an die Innenwand 4 im Bereich des Kopfabschnitts 6 ein Hakenring 8 aus dem ersten Kunststoffmaterial angeformt. Der Verschlusskörper 7 umgibt den Hakenring 8 vollständig, also auch dessen Hinterschneidungsbereiche, so dass der Hakenring 8 einen sichernden Formschlussbeitrag, was die Verbindung des Verschlusskörpers 7 mit dem Grundkörper 2 angeht, liefert. Das erste und das zweite Kunststoffmaterial sind so aufeinander abgestimmt, dass eine Haftverbindung zwischen dem Grundkörper 2 und dem Verschlusskörper 7 vorliegt, so dass der Hakenring 8 nicht zwingend erforderlich ist und lediglich eine zusätzliche Sicherung darstellt.

Der Verschlusskörper 7 hat Durchstecheigenschaften. Diese entsprechenden Anforderungen nach der DIN-Norm 58362 und 58363-15. Es ist daher möglich, den Verschlusskörper längs einer Längsachse 9 des Grundkörpers 2 mit einer Injektionsnadel vollständig durchzustechen, wobei nach dem Herausziehen der Injektionsnadel aus dem Verschlusskörper 7 letzterer die Zugangsöffnung 3 wieder vollkommen dicht verschließt.

Die Innenwand 4 der Zugangsöffnung 3 hat dort, wo der Verschlusskörper 7 an dieser anliegt, also in einem in der Fig. 3 mit A bezeichneten Bereich, einen im Wesentlichen konstanten Querschnitt. Der Hakenring 8 verringert den Querschnitt der Innenwand 4 nur geringfügig und in einem verglichen mit der axialen Abmessung A geringen Bruchteil, so dass die Querschnittsverengung des Hakenrings 8 nicht ins Gewicht fällt. Entsprechende Formschluss-Sicherungskörper sind im Verbindungsbereich des Grundkörpers 2 mit dem Verschlusskörper 7 also zulässig, ohne dass sich hierdurch eine Abweichung von einem im Wesentlichen konstanten Querschnitt im Sinne der vorliegenden Anmeldung ergeben würde.

Der Injektionsport 1, also der Grundkörper 2 zusammen mit dem Verschlusskörper 7, ist als Zweikomponenten-Spritzgussteil gefertigt. Grundsätzlich ist es möglich, zum Beispiel eine Kunststoffbeschichtung des Hülsenabschnitts 5 des Grundkörpers 2 vorzusehen, so dass der Injektionsport 1 insgesamt also auch als Mehrkomponenten-Spritzgussteil gefertigt sein kann.

Fig. 3 zeigt eine erfindungsgemäße medizinische Baugruppe, bei der der Injektionsport 1 zum Einsatz kommt. Die medizinische Baugruppe weist neben dem Injektionsport 1 noch einen Schlauchabschnitt 10 aus einem dritten Kunststoffmaterial auf aus einem Acetat- oder Acrylat-Polymer, zum Beispiel einem Ethylen-Methyl-Acrylat- oder Ethylen-Methylmethacrylat-Copolymer (EMA) oder EVA (Ethylvinylacetat). Der Schlauchabschnitt 10 ist auf den Hülsenabschnitt 5 des Grundkörpers 2 aufgeschoben. Ein dem Kopfabschnitt 6 des Grundkörpers 2 zugewandter Endbereich des Schlauchabschnitts 10 ist mit dem Hülsenabschnitt 5 über einen Verbindungsbereich 11 haftend und dicht verbunden. Das erste Kunststoffmaterial des Grundkörpers 2 und das dritte Kunststoffmaterial des Schlauchabschnitts 10 sind so aufeinander abgestimmt, dass eine Haftverbindung zwischen dem Grundkörper 2 und dem Schlauchabschnitt 10 durch Erwärmung der medizinischen Baugruppe auf eine Temperatur, die geringer ist als 160°C, entsteht. Diese Erwärmung findet bei der für die medizinische Baugruppe erforderlichen Dampfsterilisation statt. Die Haftung über den Verbindungsbereich 11 entsteht zum Beispiel durch eine 20-minütige Erwärmung bei 121°C oder durch eine 10-minütige Erwärmung bei 134°C.

Nicht erfindungsgemäße Materialbeispiele für das erste Kunststoffmaterial sind Polycarbonat (PC), Acrylnitril-Butadien-Styrol (ABS, MABS), weitere Polyolefine, zum Beispiel PE (Polyethylen), olefinische Copolymere, Polyamid (PA) sowie Abmischungen zwischen den im Zusammenhang mit dem ersten Kunststoffmaterial aufgeführten Materialien.

Nicht erfindungsgemäße Materialbeispiele für das zweite Kunststoffmaterial sind Polyurethan (PUR), elastomermodizifierte Polypropylene (EMPP), Styrolblockcopolymere (SBS, SEBS), Polyether-blockamide (PEBA), Ethylvinylacetat (EVA) und Silikon.

Je höher der PP-Anteil an der PP/COP-Abmischung ist, desto besser haftet der Verschlusskörper 7 an der Innenwand der Zugangsöffnung 3. Je höher der COP-Anteil der PP/COP-Abmischung ist, desto besser haftet der Schlauchabschnitt 10 über den Verbindungsbereich 11 am Hülsenabschnitt 5.

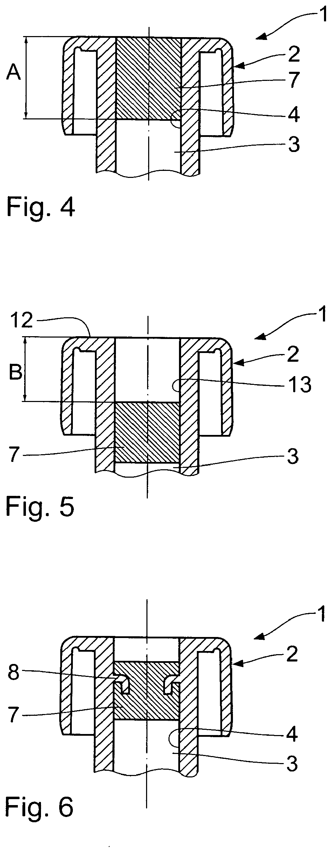

Weitere Ausführungsbeispiele der Geometrie der Verbindung des Verschlusskörpers 7 am Grundkörper 2 zeigen die Fig. 4 bis 6. Komponenten, die denjenigen entsprechen, die vorstehend schon unter Bezugnahme auf die Fig. 1 bis 3 erläutert wurden, tragen die gleichen Bezugsziffern und werden nicht nochmals im Einzelnen diskutiert.

Beim Injektionsport 1 nach Fig. 4 hat die Innenwand 4 der Zugangsöffnung 3 dort, wo der Verschlusskörper 7 an dieser anliegt, eine absolut gleichbleibende Querschnittsform, die sich über den Bereich A also nicht ändert.

Eine Formschluss-Sicherungskomponente wie der Hakenring 8 der Ausführung nach den Fig. 1 bis 3 fehlt bei der Ausführung nach Fig. 4. Bei der Ausführung nach Fig. 5 ist der Verschlusskörper 7 gegenüber einer Stirnwand 12 des Grundkörpers 2 um einem Abstand B ins Innere des Grundkörpers 2 versetzt angeordnet. Oberhalb des Verschlusskörpers 7 liegt daher ein Rücksprungbereich 13 vor, der gleichzeitig die Ausmündung der Zugangsöffnung 3 nach außen darstellt. Der Rücksprungbereich 13 verhindert beim Aufsetzen zum Beispiel einer Injektionsnadel zum Durchstechen des Verschlusskörpers 7 ein Abrutschen der Nadel vom Injektionsport 1.

Beim Injektionsport 1 nach Fig. 6 hat die Zugangsöffnung 3 dort, wo der Verschlusskörper 7 an der Innenwand 4 anliegt, einen gegenüber der restlichen Zugangsöffnung 3 vergrößerten Querschnitt. Dies verbessert die Durchstecheigenschaften des Verschlusskörpers 7, so dass dort eine gewisse Querschnittsverengung durch den Einsatz des Hakenrings 8 toleriert werden kann.

Weitere Anwendungen des medizinischen Arbeitsmittels anstelle eines Injektionsports sind die Anwendung als Zuspritzstelle für Infusions- und Dialysegeräte, als urologischer Stufenkonnektor bzw. urologischer Stufenkegel und als Verschlusskappe mit Septum.

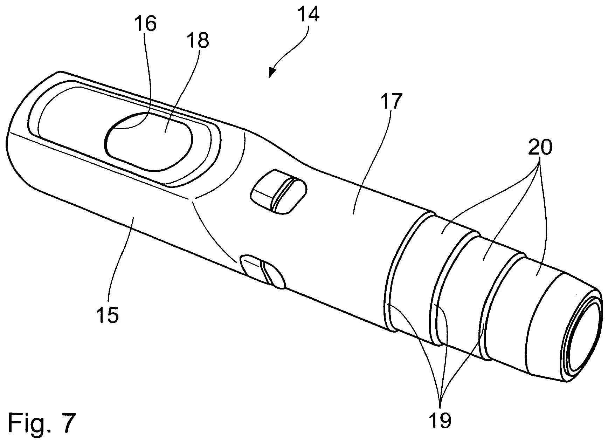

Fig. 7 zeigt eine Ausführung eines erfindungsgemäßen medizinischen Arbeitsmittels als Stufenkonnektor 14. Dieser hat einen Grundkörper 15 mit einer Zugangsöffnung 16 und einen Hülsenabschnitt 17. Der Grundkörper 15 ist aus einem einheitlichen ersten Kunststoffmaterial, wie vorstehend im Zusammenhang mit dem Injektionsport 1 beschrieben. Der Grundkörper 15 ist aus einer Mischung aus Polypropylen und einem Cycloolefin-Copolymer hergestellt, wie vorstehend im Zusammenhang mit den Ausführungen nach den Fig. 1 bis 6 erläutert. Der Stufenkonnektor 14 dient zur Verbindung von Schlauchabschnitten insbesondere mit unterschiedlichen Durchmessern.

Die Zugangsöffnung 16 wird verschlossen von einem Verschlusskörper 18 aus einem einheitlichen zweiten Kunststoffmaterial. Hierbei handelt es sich um eines der Materialien, die vorstehend schon im Zusammenhang mit dem medizinischen Arbeitsmittel nach den Fig. 1 bis 6 angegeben wurden. Das Kunststoffmaterial des Verschlusskörpers 18 hat eine im Vergleich zum Kunststoffmaterial des Grundkörpers 15 geringere Shorehärte. Der Stufenkonnektor 14 ist zur Verbindung mit einem Verbindungsabschnitt in Form eines nicht dargestellten Schlauches aus einem dritten Kunststoffmaterial vorbereitet. Beim mit dem Stufenkonnektor 14 verbundenen Schlauchabschnitt handelt es sich insbesondere um einen Katheterschlauch. Der Schlauch kann auf den Hülsenabschnitt 17 bis auf eine der dort eingearbeiteten Stufen 19, je nach dem Innendurchmesser des Schlauchs, aufgeschoben werden. Über einen Verbindungsbereich 20, der der jeweiligen Stufe 19, an der der Schlauch stirnseitig anliegt, benachbart ist, ist der Schlauch in der aufgeschobenen Stellung endseitig mit dem Grundkörper 15 des Stufenkonnektors 14 haftend und dicht verbunden. Diese Verbindung kann beispielsweise durch Erwärmen des Schlauchs erfolgen.

Der Stufenkonnektor 14 ist als Mehrkomponenten-Spritzgußteil gefertigt, wobei der Grundkörper 15 eine erste Komponente und der Verschlusskörper 18 eine zweite Komponente des Mehrkomponenten-Spritzgußteils darstellt. Die beiden Kunststoffmaterialien des Grundkörpers 15 einerseits und des Verschlusskörpers 18 andererseits sind so aufeinander abgestimmt, dass bei in die Zugangsöffnung 16 durch den Mehrkomponenten-Spritzguß eingespritzten Verschlusskörper 18 eine Haftverbindung zwischen dem Grundkörper 15 und dem Verschlusskörper 18 vorliegt.

Der Verschlusskörper 18 hat Durchstecheigenschaften, wie vorstehend im Zusammenhang mit dem Verschlusskörper 7 der Ausführungen nach den Fig. 1 bis 6 erläutert.

Der Verschlusskörper 18 kann zur Verbesserung des Anhaftens in der Zugangsöffnung 16 durch einen zusätzlichen Formschluss in der Zugangsöffnung 16 gehalten sein, wie dies vorstehend insbesondere im Zusammenhang mit den Fig. 2, 3 und 6 erläutert wurde.

Die Funktion des Verschlusskörpers 18 entspricht derjenigen, die vorstehend im Zusammenhang mit dem Verschlusskörper 7 erläutert wurde. Über ein Durchstechen des Verschlusskörpers 18 mit einer Injektionsnadel kann in montiertem Zustand des Stufenkonnektors 14 beispielsweise ein Medikament in den vom Stufenkonnektor 14 gebildeten Versorgungs-Bahnabschnitt eingeleitet werden. Der Stufenkonnektor 14 kann beispielsweise einen Luer-Lock-Ansatz haben.

## Patentansprüche

1. Medizinische Baugruppe
- mit einem medizinischen Arbeitsmittel (1; 14),
-- mit einem eine Zugangsöffnung (3; 16) und einen Hülsenabschnitt (5; 17) aufweisenden Grundkörper (2; 15) aus einem einheitlichen ersten Kunststoffmaterial,
-- mit einem die Zugangsöffnung (3; 16) verschließenden Verschlusskörper (7; 18) aus einem einheitlichen zweiten Kunststoffmaterial mit im Vergleich zum ersten Kunststoffmaterial geringerer Shorehärte,
-- wobei das Arbeitsmittel (1; 14) als Mehrkomponenten-Spritzgussteil gefertigt ist,
-- wobei das erste und das zweite Kunststoffmaterial so aufeinander abgestimmt sind, dass eine Haftverbindung zwischen dem Grundkörper (2; 15) und dem Verschlusskörper (7; 18) vorliegt,
-- wobei das zweite Kunststoffmaterial Durchstecheigenschaften aufweist und ein thermoplastisches Elastomer (TPE) ist,
- mit einem Verbindungsabschnitt (10) aus einem dritten Kunststoffmaterial, der auf den Hülsenabschnitt (5) des Grundkörpers (2) aufgeschoben und endseitig mit diesem über einen Verbindungsbereich (11) haftend und dicht verbunden ist,
**dadurch gekennzeichnet, dass**
- das erste Kunststoffmaterial so auf das dritte Kunststoffmaterial abgestimmt ist, dass eine Haftverbindung zwischen dem Grundkörper (2) und dem Verbindungsabschnitt (10) durch Erwärmung von diesen auf eine Temperatur unter 160° C entsteht,
- das erste Kunststoffmaterial eine Mischung aus Polypropylen (PP) und einem Cycloolefin-Polymer (COP) ist,
- ein Gewichtsprozentverhältnis PP/COP zwischen 90:10 und 10:90 ist,
- der Verbindungsabschnitt (10) ein Acetat- oder Acrylat-Polymer umfasst.

2. Medizinische Baugruppe nach Anspruch1, **dadurch gekennzeichnet dass**, das Gewichtsprozentverhältnis PP/COP zwischen 80:20 und 20:80, mehr bevorzugt zwischen 70:30 und 30:70, noch mehr bevorzugt zwischen 60:40 und 40:60 und noch mehr bevorzugt von 50:50 ist.

3. Medizinische Baugruppe nach einem der Ansprüche 1 bis 2, **dadurch gekennzeichnet, dass** eine Innenwand (4) der Zugangsöffnung (3) dort, wo der Verschlusskörper (7) an dieser anliegt, einen im Wesentlichen konstanten Querschnitt aufweist.

4. Medizinische Baugruppe nach Anspruch 3, **dadurch gekennzeichnet, dass** die Innenwand (4) der Zugangsöffnung (3) dort, wo der Verschlusskörper (7) an dieser anliegt, eine absolut gleich bleibende Querschnittsform aufweist.

5. Medizinische Baugruppenach einem der Ansprüche 1 bis 4, **dadurch gekennzeichnet, dass** der Verschlusskörper (7) gegenüber einer Stirnwand (12) des Grundkörpers (2), aus der die Zugangsöffnung (3) ausmündet, nach innen versetzt angeordnet ist.

## Claims

1. Medical assembly
- having a medical tool (1; 14),
- having a main body (2; 15) that has an access opening (3; 16) and a sleeve portion (5; 17) and is made of a unitary first plastics material,
- having a closing body (7; 18) that closes the access opening (3; 16) and is made of a unitary second plastics material with a lower Shore hardness than the first plastics material,
- wherein the tool (1; 14) is manufactured as a multicomponent injection-moulded part,
- wherein the first and the second plastics material are coordinated with one another such that there is an adhesive bond between the main body (2; 15) and the closing body (7; 18),
- wherein the second plastics material has piercing properties and is a thermoplastic elastomer (TPE),
- having a connecting portion (10) made of a third plastics material that has been pushed onto the sleeve portion (5) of the main body (2) and is connected thereto in an adhering and impermeable manner at its end via a connecting region (11),
**characterized in that**
- the first plastics material is coordinated with the third plastics material such that an adhesive bond between the main body (2) and the connecting portion (10) arises when they are heated to a temperature of less than 160°C,
- the first plastics material is a mixture of polypropylene (PP) and a cycloolefin polymer (COP),
- a weight percentage ratio PP/COP is between 90:10 and 10:90,
- the connecting portion (10) comprises an acetate polymer or acrylate polymer.

2. Medical assembly according to Claim 1, **characterized in that** the weight percentage ratio PP/COP is between 80:20 and 20:80, more preferably between 70:30 and 30:70, even more preferably between 60:40 and 40:60, and even more preferably 50:50.

3. Medical assembly according to either of Claims 1 and 2, **characterized in that** an inner wall (4) of the access opening (3) has a substantially constant cross section where the closing body (7) bears against it.

4. Medical assembly according to Claim 3, **characterized in that** the inner wall (4) of the access opening (3) has an absolutely unvarying cross-sectional shape where the closing body (7) bears against it.

5. Medical assembly according to one of Claims 1 to 4, **characterized in that** the closing body (7) is arranged in a manner offset inwardly with respect to an end wall (12) of the main body (2), out of which the access opening (3) leads.

## Revendications

1. Ensemble médical comprenant
- un article médical (1 ; 14) comprenant,
-- un corps de base (2 ; 15) en une première matière plastique uniforme comportant une ouverture d'accès (3 ; 16) et une section de manchon (5 ; 17),
-- un corps de fermeture (7 ; 18) en une deuxième matière plastique uniforme présentant une dureté Shore inférieure à celle de la première matière plastique, lequel corps ferme l'ouverture d'accès (3 ; 16),
-- l'article (1 ; 14) étant réalisé comme une pièce moulée par injection à plusieurs composants,
-- les première et deuxième matières plastiques étant adaptées l'une à l'autre de sorte qu'il existe une liaison adhésive entre le corps de base (2 ; 15) et le corps de fermeture (7 ; 18),
-- la deuxième matière plastique présentant des propriétés de perforation et étant un élastomère thermoplastique (TPE),
- comprenant une section de liaison (10) en une troisième matière plastique, qui est enfilée sur la section de manchon (5) du corps de base (2) et qui est reliée de manière adhésive et étanche à ses extrémités par une zone de raccordement (11), **caractérisé en ce que**
- la première matière plastique est adaptée à la troisième matière plastique, de sorte qu'une liaison adhésive se forme entre le corps de base (2) et la section de liaison (10) en chauffant ceux-ci à une température inférieure à 160 °C,
- la première matière plastique est un mélange de polypropylène (PP) et d'un polymère cyclooléfinique (COP),
- un rapport de pourcentage en poids PP/COP est compris entre 90 : 10 et 10 :90,
- la section de liaison (10) comprend un polymère d'acétate ou d'acrylate.

2. Ensemble médical selon la revendication 1, **caractérisé en ce que** le rapport de pourcentage en poids PP/COP est compris entre 80 :20 et 20 :80, plus préférablement entre 70 :30 et 30 :70, mieux encore entre 60 :40 et 40 :60 et est mieux encore de 50 :50.

3. Ensemble médical selon l'une des revendications 1 à 2, **caractérisé en ce qu'**une paroi intérieure (4) de l'ouverture d'accès (3) présente une section transversale sensiblement constante à l'endroit où le corps de fermeture (7) repose sur elle.

4. Ensemble médical selon la revendication 3, **caractérisé en ce que** la paroi intérieure (4) de l'ouverture d'accès (3) présente une forme de section transversale absolument constante à l'endroit où le corps de fermeture (7) repose sur elle.

5. Ensemble médical selon l'une des revendications 1 à 4, **caractérisé en ce que** le corps de fermeture (7) est disposé de manière décalée vers l'intérieur par rapport à une paroi frontale (12) du corps de base (2), à partir de laquelle l'ouverture d'accès (3) débouche.
